# EUROPÄISCHE PATENTSCHRIFT

(11) **EP 3 275 558 B1**
(45) Veröffentlichungstag und Bekanntmachung des Hinweises auf die Patenterteilung: **20.03.2019**
(21) Anmeldenummer: 16181527.9
(22) Anmeldetag: 27.07.2016
(51) Int. Cl.: B05B 15/00, B65D 83/14, B05B 1/14, B65D 83/30

(54) **SPRÜHANORDNUNG SOWIE SPENDER MIT EINER SOLCHEN SPRÜHANORDNUNG UND AUSTRAGKOPF FÜR EINEN SOLCHEN SPENDER**
SPRAY ASSEMBLY AND DISPENSER WITH SUCH A SPRAY ASSEMBLY, AND APPLICATOR HEAD FOR SUCH A DISPENSER
ENSEMBLE DE PULVERISATION ET DISTRIBUTEUR COMPRENANT UN TEL ENSEMBLE DE PULVERISATION ET TETE DISTRIBUTRICE POUR UN TEL DISTRIBUTEUR

(43) Veröffentlichungstag der Anmeldung: 31.01.2018
(73) Patentinhaber: Aptar Radolfzell GmbH, 78315 Radolfzell (DE)
(72) Erfinder: Greiner-Perth, Jürgen, 78244 Gottmadingen (DE); Krampen, Gerald, 78315 Radolfzell (DE)
(74) Vertreter: Patentanwaltskanzlei Cartagena

(56) Entgegenhaltungen:
- EP-A1- 2 484 199
- EP-A2- 2 767 346
- WO-A1-2012/007315
- WO-A1-2013/064690
- DE-A1-102011 011 502
- FR-A1- 2 915 470
- US-A- 3 756 472

## Beschreibung

### ANWENDUNGSGEBIET UND STAND DER TECHNIK

Die Erfindung betrifft eine Sprühanordnung, insbesondere zum Austrag von pharmazeutischen oder kosmetischen Flüssigkeiten nach dem Oberbegriff von Anspruch 1. Sie betrifft weiterhin einen Austragkopf für einen Spender zum Austrag von pharmazeutischen oder kosmetischen Flüssigkeiten nach dem Oberbegriff von Anspruch 4 mit einer Sprühanordnung sowie einen Spender nach dem Oberbegriff von Anspruch 12 zum Austrag von pharmazeutischen oder kosmetischen Flüssigkeiten mit einem Austragkopf.

Eine gattungsgemäße Sprühanordnung ist beispielsweise aus der FR2915470 oder aus der WO 2015/194962 A1 des Anmelders Medspray B.V. bekannt. Die dort offenbarte Sprühanordnung ist in Art einer hülsenartigen Filter- und Düseneinheit gestaltet. Innerhalb eines Hülsenbauteils befinden sich neben einer Düsenplatte auch ein oder mehrere Filter. Die Einheit kann in der aus der genannten Schrift bekannten Form gut während folgenden Montageschritten gehandhabt werden.

Es hat sich jedoch herausgestellt, dass trotz der Filtergestaltung bei lang anhaltenden Austrägen von einigen Sekunden oder Minuten Länge der vorgesehene Tiefenfilter sich zusetzt.

### AUFGABE UND LÖSUNG

Aufgabe der Erfindung ist es, eine Sprühanordnung sowie einen Austragkopf und einen Spender mit einer solchen Sprühanordnung zur Verfügung zu stellen, wobei die Sprühanordnung in der Lage ist, auch lang anhaltende Sprühvorgänge bei gleichbleibend gutem Sprühbild zu ermöglichen.

Eine erfindungsgemäße Sprühanordnung umfasst einen Zuführkanal zur Zuführung von Flüssigkeit zum Zwecke des Versprühens sowie eine Düsenplatte, durch die hindurch Flüssigkeit vom Zuführkanal in eine umgebende Atmosphäre ausgetragen werden kann. Diese Düsenplatte, bei der es sich vorzugsweise um eine Düsenplatte aus einem Siliziumwerkstoff handelt, weist eine Vielzahl von Düsenöffnungen auf, deren Durchmesser maximal 15 µm beträgt und die gemeinsam eine Düsenöffnungsfläche der Düsenplatte definieren.

Die Sprühanordnung umfasst in einem Strömungspfad zwischen dem Zuführkanal und der Düsenplatte eine Filtermembran, deren Trenngrenze kleiner als der Durchmesser der Düsenöffnungen ist und deren im Strömungspfad gelegene wirksame Filterfläche mindestens Faktor 20 größer ist als die Düsenöffnungsfläche.

Bei einer erfindungsgemäßen Sprühanordnung ist demnach vorgesehen, statt des bislang benutzten Tiefenfilters, der bei der genannten Gestaltung des Stands der Technik in das Hülsenbauteil eingesetzt ist und daher eine identische oder nur geringfügig größere Filterfläche als die Düsenöffnungsfläche aufweist, eine Filtermembran zu verwenden und diese erheblich größer als die Düsenöffnungsfläche zu gestalten. Unter der Düsenöffnungsfläche im Sinne der Erfindung wird jene Fläche verstanden, auf der die Düsenöffnungen vorgesehen sind. Es handelt sich um das kleinste denkbare Polygon, innerhalb dessen alle Düsenöffnungen angeordnet sind.

Die Verwendung einer großflächigen Filtermembran statt eines bezüglich des Durchmessers geringen Tiefenfilters hat sich insbesondere bei lang anhaltenden durchgehenden Sprühvorgängen als bessere Lösung herausgestellt. Der bisher verwendete Tiefenfilter, der gemeinsam mit der Düsenplatte in einem zylindrischen Hülsenbauteil angeordnet ist, ist zwar durch seine Tiefe grundsätzlich ebenfalls geeignet, ähnliche Flüssigkeitsmengen wie ein deutlich größerer Membranfilter zu reinigen. Es wurde jedoch festgestellt, dass der Tiefenfilter bei lang anhaltender und ununterbrochener Flüssigkeitsbeaufschlagung schneller sein Filterverhalten ändert als die vorgeschlagene Filtermembran.

Die Filtermembran ist mit einer Trenngrenze versehen, die kleiner als der Durchmesser der Düsenöffnungen ist. Bei Düsenöffnungen mit einem geringsten Durchmesser von 15 µm liegt die Trenngrenze demzufolge unterhalb dieser 15 µm. Vorzugsweise liegt die Trenngrenze mindestens 20 % unter dem mittleren Durchmesser der Düsenöffnungen. Unter der Trenngrenze der Filtermembran im Sinne dieser Erfindung wird jener Partikeldurchmesser verstanden, bezüglich dessen mindestens 85 % der entsprechenden Partikel bei einem auf der Anströmseite anliegenden Überdruck von 0,1 bar von der Filtermembran herausgefiltert werden. Diese Werte beziehen sich auf eine zuvor unbenutzte Filtermembran, also den Neuzustand.

Im Zusammenhang mit dieser Beschreibung ist unter dem Durchmesser der Düsenöffnungen der jeweils geringste Durchmesser zu verstehen, der bei der Durchquerung der Düsenöffnungen von der Flüssigkeit passiert werden muss. Die im Zusammenhang mit der erfindungsgemäßen Sprühanordnung vorzugsweise verwendeten Düsenplatten aus Siliziumwerkstoff gestatten eine sehr feine Vernebelung der Flüssigkeit, ohne dass es hierfür über die Düsenplatte hinaus weiterer zerstäubungsbegünstigender Geometrien bedarf. Die Herstellung erfolgt vorzugsweise auf Basis eines Siliziumwafers, in den die Düsenöffnungen mittels eines Ätzverfahrens eingebracht sind.

Die Anzahl der Düsenöffnungen hängt vom Anwendungsfall ab. Die Düsenplatten gemäß dieser Erfindung weisen vorzugsweise mindestens zehn Düsenöffnungen auf. Düsenplatten für die hier insbesondere betroffenen Inhalationseinrichtungen weisen vorzugsweise zwischen 10 und 200, insbesondere vorzugsweise zwischen 20 und 100 Düsenöffnungen auf. Bei kosmetischen Anwendungen, beispielsweise beim Austrag von Parfum, Deodorant oder auch Haarspray können auch Düsenplatten mit deutlich mehr Düsenöffnungen zweckmäßig sein, um einen hohen Gesamtvolumenstrom zu ermöglichen.

Wie bereits erläutert, bilden die Düsenöffnungen durch ihre Anordnung eine Düsenöffnungsfläche. Diese beträgt vorzugsweise weniger als 4 mm², insbesondere vorzugsweise weniger als 1 mm². Diese enge Anordnung der Düsenöffnungen ist im Hinblick auf einen gut orientierbaren Gesamtsprühstrahl von Vorteil. Demgegenüber ist erfindungsgemäß die wirksame Filterfläche, also der Anteil der Filtermembran, der einbaubedingt angeströmt werden kann, mindestens um den Faktor 20 größer. Im Falle einer Düsenöffnungsfläche von etwa 1 mm² beträgt die wirksame Filterfläche somit erfindungsgemäß mindestens 20 mm². Vorzugsweise wird noch ein größerer Faktor gewählt, beispielsweise von 50, 100 oder gar 200. Eine besonders bevorzugte Gestaltung sieht eine Düsenöffnungsfläche zwischen 0,6 mm² und 1 mm² sowie eine wirksame Filterfläche von mindestens 100 mm² vor.

Die Trenngrenze der Filtermembran ist insbesondere in Relation zum Durchmesser der Düsenöffnungen zu wählen, jedoch auch in Abhängigkeit davon, welche Schwebstoffe oder Kristalle typbedingt in der Flüssigkeit zu erwarten sind. Der bereits genannte Durchmesser der Düsenöffnungen von 15 µm ist vergleichsweise groß. Um einen feineren Nebel zu bewirken, wie er insbesondere im Bereich von Inhalatoren von Vorteil ist, sind die Düsenöffnungen vorzugsweise nicht größer als 5 µm. Je nach Anwendungszweck sind daher Filtermembranen mit einer Trenngrenze von maximal 5 µm oder gar maximal 2 µm, vorzugsweise maximal 1 µm oder sogar maximal 0,2 µm von Vorteil.

Die Düsenplatte und die Filtermembran sind vorzugsweise parallel zueinander angeordnet und insbesondere vorzugsweise mindestens 3 mm voneinander beabstandet, um der durch die Filtermembran gelangten Flüssigkeit Gelegenheit zur Homogenisierung des Flüssigkeitsstroms zu geben.

Die Dicke der Filtermembran beträgt vorzugsweise maximal 10 % der minimalen Erstreckung der wirksamen Filterfläche in der Erstreckungsebene der Filterfläche. Bevorzugte Membranen sind jedoch nochmals dünner und weisen vorzugsweise eine Dicke von maximal 3 % der minimalen Erstreckung der wirksamen Filterfläche auf. Nicht zur Dicke der Membran zählen für die Filterung unwirksame und mit der Membran gemeinsam auslenkbare Stützschichten.

Als besonders bevorzugtes Material für die Filtermembran wird PES (Polyethersulfon) angesehen. Es sind jedoch auch andere Materialien zweckmäßig verwendbar. Hierzu zählen: Polysulfon, Polyethersulfon, Cellulose, Celluloseester, Silikone, Polyamid, Polyamidimid, Polyamid Harnstoff, Polycarbonat, Keramik, Edelstahl, Silber, Silizium, Zeolithe, Polyacrylnitril, Polyethylen, Polypropylen, Polytetrafluorethylen, Polyvinylidenfluorid, Polyvinylchlorid, Polypiperazinamid.

Der erfindungsgemäße Austragkopf weist eine Sprühanordnung vorbeschriebener Art auf.

Der erfindungsgemäße Austragkopf zeichnet sich dadurch aus, dass er üblicherweise ein Außengehäuse aufweist, welches durch einen Flüssigkeitsauslass durchdrungen ist, so dass innerhalb des Austragkopfes zugeführte Flüssigkeit an der im Flüssigkeitsauslass angeordneten Düsenplatte vernebelt werden kann und so in eine umgebende Atmosphäre ausgetragen werden kann. Ein solcher Austragkopf wird bestimmungsgemäß in Flüssigkeitsspendern verwendet, die zusätzlich auch das erforderliche Flüssigkeitsreservoir beinhalten. Bevorzugterweise ist der Austragkopf gegenüber dem Flüssigkeitsreservoir beweglich, um beispielsweise über eine Niederdrückbewegung oder eine Drehbewegung den Austrag manuell initiieren zu können.

Die Düsenplatte und die Filtermembran dieses Austragkopfes oder einer erfindungsgemäßen Sprühanordnung sind bei einer Ausgestaltung in einer gemeinsamen Baueinheit zusammengefasst, die ein Hülsenbauteil umfasst, welches von einem Austragkanal durchdrungen ist, innerhalb dessen die Düsenplatte und die Filtermembran angeordnet sind.

Diese Maßnahme dient dem Zweck, die Montage eines solchen Austragkopfes einfach zu gestalten. Durch die Integration der Düsenplatte und der Filtermembran in eine gemeinsame Baueinheit, deren tragendes Bauteil das genannte Hülsenbauteil ist, kann durch die Einbringung eines einzigen Bauteils in den Austragkopf die Filter- und Zerstäubungsfunktionalität eingebracht werden. Das Hülsenbauteil weist aufgrund der bestimmungsgemäß großen Unterschiede zwischen der Düsenöffnungsfläche einerseits und der erforderlichen wirksamen Filterfläche andererseits vorzugsweise einen Durchmessersprung auf. In einem ersten zylindrischen Abschnitt ist die Filtermembran vorgesehen. In einem zweiten zylindrischen Abschnitt, dessen Durchmesser vorzugsweise weniger als 50 % des ersten Abschnitts beträgt, ist die Filtermembran vorgesehen. Der Abstand zwischen der Filtermembran und dem Durchmessersprung des Hülsenbauteils beträgt vorzugsweise mindestens 2 mm.

Die Düsenplatte ist bei einer anderen Ausgestaltung Teil einer Düseneinheit, die ein Hülsenbauteil umfasst, welches von einem Austragkanal durchdrungen ist, innerhalb dessen die Düsenplatte angeordnet ist. Bei dieser Ausgestaltung ist weiter vorgesehen, dass die Filtermembran Teil einer von der Düseneinheit separaten Filtereinheit ist, die ein ebenfalls Hülsenbauteil umfasst, welches von einem Austragkanal durchdrungen ist, innerhalb dessen die Filtermembran angeordnet ist.

Bei dieser alternativen Gestaltung sind zwei Hülsenbauteile vorgesehen, die grundsätzlich separat handhabbar sind. Dabei ist das Hülsenbauteil der Düseneinheit vorzugsweise mit einem deutlich geringeren Außendurchmesser vorgesehen, der insbesondere vorzugsweise maximal 50 % des Außendurchmessers des Hülsenbauteils der Filtereinheit aufweist. Eine solche Gestaltung ist von Vorteil, wenn in Abhängigkeit der auszutragenden Flüssigkeit und der zu erwartenden kontinuierlichen Austragsdauer variable Konfigurationen möglich bleiben sollen, also beispielsweise die Verwendung identischer Düseneinheiten je nach Anwendungszweck mit unterschiedlichen Filtereinheiten.

Die Düseneinheit und die Filtereinheit verfügen bei einer Ausgestaltung über zusammenwirkende Kopplungsabschnitte, so dass sie zu einer gemeinsam handhabbaren Düsen- und Filtereinheit zusammengefügt werden können. Die separat handhabbaren Einheiten können so in einem vorbereitenden Montageschritt zusammengefügt werden. Dies kann insbesondere zweckmäßig sein, wenn die Düseneinheit und die Filtereinheit flexibel kombinierbar sein sollen, jedoch an einem Montagestandort bereits in Abhängigkeit des spezifischen Anwendungszwecks ausgewählt und zusammengefügt werden und dann an einem anderen Montageort gemeinsam in einen Austragkopf eingefügt werden. Insbesondere können die Kopplungsabschnitte an der Düseneinheit und der Filtereinheit in Form formschlüssig einen Presssitz bildender Außen- und Innenkonturen vorliegen. Die Düseneinheit und die Filtereinheit werden also bei der Montage ineinandergesteckt, um eine gemeinsame Düsen- und Filtereinheit zu bilden. Eine solche Filtereinheit kann dann als Ganzes in einen hierfür vorgesehenen Aufnahmebereich eines Gehäuses des Austragkopfes eingefügt werden.

Der Austragkopf verfügt bei einer Ausgestaltung über ein Gehäuseteil mit Aufnahmebereichen für die Düseneinheit und die Filtereinheit. Diese Aufnahmebereiche sind zum Einsetzen der Düseneinheit und der Filtereinheit in übereinstimmender Montagerichtung ausgebildet.

Bei einer solchen Gestaltung sind somit im genannten Gehäuseteil zwei getrennte Aufnahmebereiche vorgesehen, die einerseits der Aufnahme der Düseneinheit und andererseits der Aufnahme der Filtereinheit dienen. Die Düseneinheit und die Filtereinheit werden in ihren jeweiligen Aufnahmebereichen vorzugsweise über Presssitze gehalten. Durch die übereinstimmende Montagerichtung lässt sich ein einfacher Montagevorgang erzielen, bei der insbesondere nacheinander von oben die Düseneinheit und die Filtereinheit in ein zuvor positioniertes Gehäuseteil eingesetzt werden können.

Die Düseneinheit und die Filtereinheit sind bei einer Weiterbildung in dieses Gehäusebauteil eingesetzt, welches seinerseits mit einem zweiten Gehäusebauteil in mit der Düseneinheit und/oder der Filtereinheit übereinstimmender Montagerichtung verschlossen ist.

Bei dieser Gestaltung ist somit ein zweites Gehäusebauteil vorgesehen, welches im Zuge der Montage in gleicher Richtung wie zuvor die Düseneinheit und/oder die Filtereinheit auf das erste Gehäusebauteil aufgesetzt oder in dieses eingesetzt werden kann. Hierbei ergibt sich ein besonderer Vorteil, wenn durch dieses zweite Gehäusebauteil auch eine Ladefixierung der Düseneinheit oder der Filtereinheit in ihrem jeweiligen Aufnahmebereich erfolgt.

Zur Erzielung einer axial kurzen Filter- und Düsenanordnung ist es von Vorteil, wenn die Filtereinheit und die Düseneinheit derart aufeinander abgestimmt sind, dass eines der Hülsenbauteile, insbesondere das Hülsenbauteil der Düseneinheit, zumindest teilweise innerhalb des anderen Hülsenbauteils, vorzugsweise des Hülsenbauteils der Filtereinheit, angeordnet werden kann. In Strömungsrichtung der Flüssigkeit sind die beiden Hülsenbauteile somit gewissermaßen überlappend eingebaut. Somit kann selbst bei einer aufgrund der Flexibilität vorteilhaften Anordnung mit getrennten Filtereinheiten und Düseneinheiten der Bauraumbedarf reduziert werden.

Der Austragkopf verfügt bei einer Ausgestaltung über eine Stützstruktur stromabwärts der Filtermembran, durch die die Auslenkung der Filtermembran limitiert ist.

Eine solche Stützstruktur ist aufgrund der vergleichsweise großen Fläche der Filtermembran von Vorteil. Die Stützstruktur ist vorzugsweise Teil des ersten Gehäusebauteils und umgibt vorzugsweise das Hülsenbauteil der Düseneinheit. Die Stützstruktur kann insbesondere durch im Hinblick auf einfache Herstellbarkeit vorteilhafte mit Durchbrechungen versehene Wandungen gebildet werden, deren Stirnseiten in Richtung der Filtermembran weisen und von dieser nur gering beabstandet sind, so dass eine die Membran potenziell verletzende Auslenkung derselben verhindert wird, weil diese nach geringer Auslenkung sich an die besagten Stirnseiten anlegt.

Das Hülsenbauteil der Düseneinheit und/oder das Hülsenbauteil der Filtereinheit oder das Hülsenbauteil der gemeinsamen Filter- und Düseneinheit ist vorzugsweise als Kunststoffteil ausgebildet.

Die Filtermembran ist vorzugsweise vom Hülsenbauteil der Filtereinheit oder der gemeinsamen Filter- und Düseneinheit umspritzt.

Die Filtereinheit verfügt vorzugsweise über einen Schutzrand, der derart an die Geometrie der Filtereinheit angepasst ist, dass es bei zwei baugleichen Filtereinheiten nicht möglich ist, eine Oberfläche der Membran der einen Filtereinheit mit der anderen Filtereinheit zu berühren. Durch einen ausreichend breiten Schutzrand wird gewährleistet, dass selbst dann, wenn die Filtereinheiten als Schüttgut behandelt werden, stets nur Schutzränder mehrerer baugleicher Filtereinheiten in Kontakt miteinander gelangen, nicht jedoch die Gefahr besteht, dass der Rand einer Filtereinheit unmittelbar in Kontakt mit der Membran einer anderen Filtereinheit gelangen kann und diese so verletzen könnte.

Der erfindungsgemäße Spender weist einen Austragkopf vorbeschriebener Art auf. Dieser Spender umfasst einen Flüssigkeitsspeicherzur Lagerung von Flüssigkeit vor dem Versprühen. Der Zuführkanal des Austragkanals ist über eine Fördereinrichtung in Art einer Pumpe oder eines Auslassventils mit dem Flüssigkeitsspeicher verbunden ist.

Die Gestaltung mit einer Pumpe sieht vorzugsweise eine Kolbenpumpe vor, die durch Niederdrücken des Austragkopfes gegenüber dem Flüssigkeitsspeicher betätigt wird. Die Gestaltung mit einem Auslassventil sowie, hiermit verbunden, mit einem Druckspeicher als Flüssigkeitsspeicher, wird als bevorzugt angesehen, da sie es gestattet, insbesondere bei der Verwendung eines solchen Spenders als Inhalator einen lang anhaltenden homogenen Sprühstrahl über einige Sekunden oder gar Minuten zu erzeugen. Insbesondere in diesem Kontext ist die erfindungsgemäße Gestaltung mit großer Filtermembran von Vorteil.

Der Flüssigkeitsspeicher ist vorzugsweise zur Aufnahme von maximal 500 ml ausgelegt, vorzugsweise von maximal 250 ml.

Er ist bevorzugt gefüllt meiner einer der folgenden Flüssigkeiten zum Zwecke der Inhalation: einer salzhaltigen wässrigen Lösung oder einer wässrigen Lösung in Form einer Ringerlösung oder einer gepufferten Lösung oder einer wässrigen Lösung mit mindestens einem der Zusätze Kohlenhydrate, ätherische Öle, Menthol und Pflanzenextrakte oder einer wässrigen Lösung, enthaltend Vitamine, Spurenelemente, Mangan oder Zink, oder einer wässrigen Lösung mit mindestens einem der Zusätze aus der Gruppe umfassend Zimtöl, Teebaumöl, Salbeiöl, Thymianöl, Zitronenmelissenöl.

Der Spender ist bei einer Ausgestaltung als Inhalator ausgebildet und verfügt über ein Atemstück, welches als Mundstück oder als Atemmaske ausgestaltet ist.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt einen Flüssigkeitsspender, der als Inhalator ausgebildet ist.
Fig. 2 und 3 zeigen den Flüssigkeitsspender und dessen Austragkopf in einer geschnittenen Darstellung.
Fig. 4 zeigt die eine Filtereinheit und eine Düseneinheit des Flüssigkeitsspenders in ihrer Einbaurelativlage.
Fig. 5 zeigt die Düseneinheit in einer stirnseitigen Perspektive.
Fig. 6 zeigt die flüssigkeitsführenden Bauteile des Austragkopfes in einer Explosionsdarstellung.
Fig. 7 bis 8 zeigen in einer geschnittenen Seitenansicht die Filtereinheit und eine Düseneinheit sowie zwei alternative Gestaltungen einer Filter- und Düseneinheit.

### DETAILLIERTE BESCHREIBUNG DER AUSFÜHRUNGSBEISPIELE

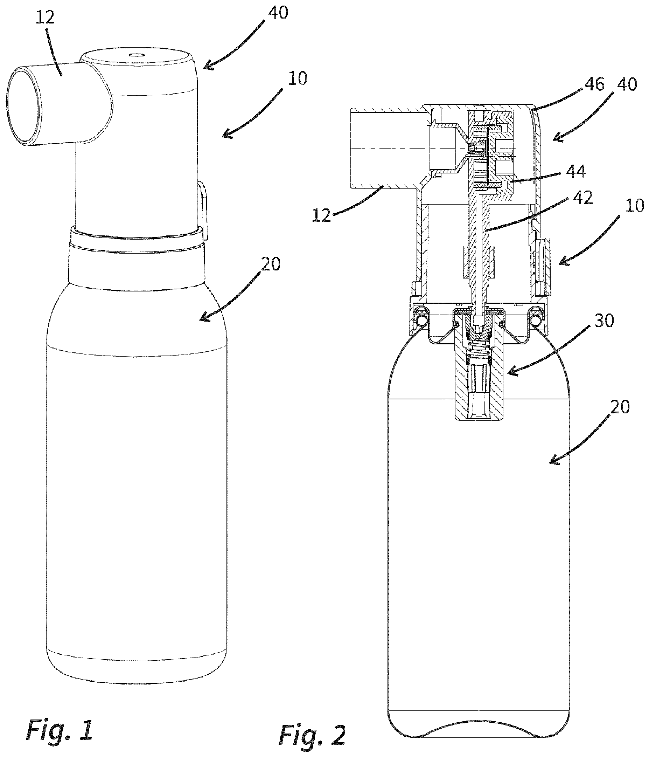

Fig. 1 zeigt einen erfindungsgemäßen Flüssigkeitsspender 10, dessen Hauptbestandteile ein als Druckspeicher ausgebildeter Flüssigkeitsspeicher 20, eine in Fig. 1 nicht dargestellte Fördereinrichtung 30 sowie ein Austragkopf 40 sind. Der Flüssigkeitsspender 10 ist als Inhalator ausgebildet und verfügt über ein Atemstück 12, welches der Benutzer mit den Lippen umschließen kann oder an das er eine Atemmaske oder dergleichen anschließen kann.

Die Flüssigkeit ist im Flüssigkeitsspeicher 20 gelagert, bis sie durch ein Öffnen der als Ventileinrichtung ausgebildeten Fördereinrichtung 30 unter Druck in den Austragkopf strömt. Hier wird die Flüssigkeit gefiltert und anschließend durch eine Düsenplatte 54 hindurch ausgetragen, wobei die Düsenplatte 54 eine Vernebelung bewirkt. Der Flüssigkeitsnebel wird in das Atemstück 12 des Austragkopfes 40 geleitet, von wo aus er unmittelbar inhaliert werden kann.

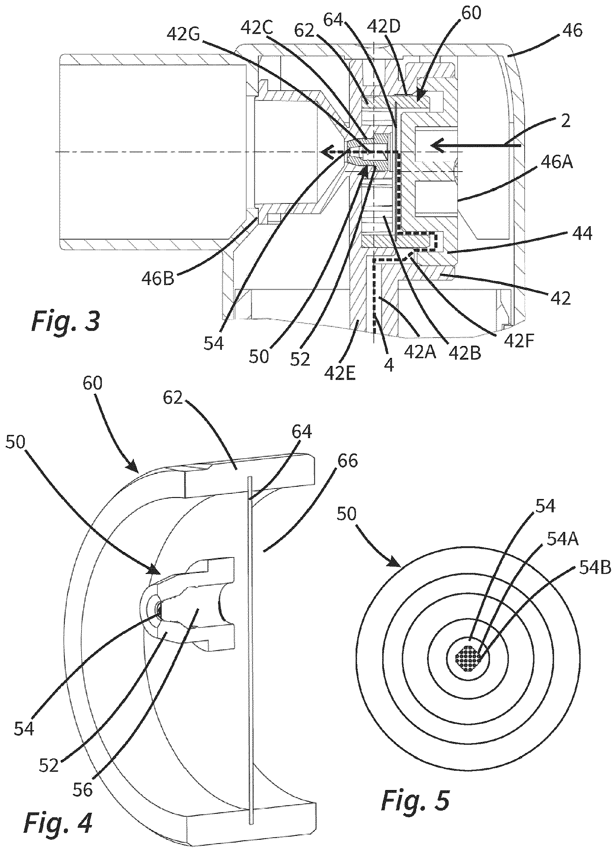

Bezug nehmend auf die Fig. 2 und 3 wird der innere Aufbau des Flüssigkeitsspenders 10 erläutert. Der Flüssigkeitsspeicher 20 ist, wie bereits beschrieben, als Druckspeicher gestaltet. Die zu inhalierende Flüssigkeit liegt hierin somit unter einem Druck vor, so dass sie beim Öffnen der als Ventileinrichtung ausgebildeten Fördereinrichtung 30 in den Austragkopf 40 einströmt, wobei hierfür der Austragkopf über einen Zuführkanal 42A verfügt. Das Schalten der Ventileinrichtung erfolgt durch eine nach oben bzw. unten gerichtete Verlagerung des Rohrabschnitts 42E, innerhalb dessen der Zuführkanal 42A vorgesehen ist. Diese Verlagerung kann beispielsweise durch eine helixförmige Drehbewegung des Austragkopfes 40 gegenüber dem Flüssigkeitsspeicher 20 oder über eine rein axiale Bewegung erzielt werden.

Bezug nehmend auf Fig. 3 ist ersichtlich, welchen Strömungspfad 4 die Flüssigkeit ausgehend vom Zuführkanal 42A bis hin in das Atemstück 12 nimmt. Am Ende des Rohrstücks 42E schließt sich ein ringförmiger Raum 42F an, der einerseits von einem Gehäusebauteil 42 gebildet wird, zu dem auch der Rohrabschnitt 42E gehört, und andererseits durch ein zweites Gehäusebauteil 44 in Art eines Stopfenbauteils begrenzt wird. Das Stopfenbauteil ist zu diesem Zweck in Montagerichtung 2 in das erste Gehäusebauteil 42 eingeschoben und kann hier über einen Presssitz gehalten werden. Aus dem genannten Ringraum 42F strömt die Flüssigkeit zur Filtermembran 64, an der Verunreinigungen wie Kristalle oder Schwebstoffe herausgefiltert werden, die jenseits der Trenngrenze von 2 µm liegen. In den sich an die Filtermembran 64 anschließenden Bereich 42G gelangt demzufolge nur Flüssigkeit, die keine so großen Verunreinigungen mehr aufweist. Diese gereinigte Flüssigkeit strömt in eine Düseneinheit 50, die über ein Hülsenbauteil 52 und eine darin eingesetzte Düsenplatte 54 verfügt. Die Düsenplatte wiederum weist 24 Düsenöffnungen 54B von jeweils 3 µm Durchmesser auf, die gemeinsam eine Düsenöffnungsfläche 54A definieren, die in den Fig. 4 und 5 verdeutlicht ist und die beispielsweise eine Größe von 0,8 mm² aufweisen kann. Beim Durchströmen der Düsenöffnungen 54B erfolgt die Vernebelung der Flüssigkeit, die somit als zur Inhalation geeigneter Inhalationsnebel das Atemstück 12 erreicht. Die feine Vernebelung führt verbunden mit der Entfernung von Verunreinigungen zu einem sehr gut für die Inhalation geeigneten Inhalationsnebel.

Bezug nehmend auf Fig. 4 ist zu ersehen, dass die Grundbauweise der Düseneinheit 50 und der Filtereinheit 60 einander ähneln. Beide weisen jeweils eine aus Kunststoff gefertigte Hülse 52, 62 auf, die jeweils von einem Austragkanal 56, 66 durchdrungen sind, in dem die Düsenplatte 54 bzw. die Filtermembran 64 angeordnet sind. Aufgrund der bestimmungsgemäß sehr unterschiedlichen Fläche, die einerseits die Düsenplatte 54 und insbesondere die Düsenöffnungsfläche 54A einnimmt und andererseits die Filtermembran 64 einnimmt, ist diese vorteilhafte Gestaltung mit zwei unterschiedlichen Einheiten 50, 60, die getrennt gehandhabt werden können, gewählt worden. Um dennoch einen in Montagerichtung 2 kompakten Aufbau zu ermöglichen, ist die Düseneinheit 50 partiell innerhalb des durch das Hülsenbauteil 62 definierten Raums angeordnet. Zudem ist vorgesehen, dass nicht nur das zweite Gehäusebauteil 44 in Art eines Stopfens, sondern auch die Düseneinheit 50 sowie die Filtereinheit 60 alle in übereinstimmender Montagerichtung 2 zusammengefügt werden können, was die Montage des Flüssigkeitsspenders 10 deutlich erleichtert. Damit auch bei je nach Anwendungsgebiet mitunter hohen Drücken das erste und das zweite Gehäusebauteil 42, 44 sich nicht voneinander trennen, agiert das Außengehäuse 46, an dem auch das Atemstück 12 vorgesehen ist, in Art einer Spange. Die nach innen weisenden Flächen 46A, 46B halten die beiden Gehäusebauteile 42, 44 im montiertem Zustand zusammen. Dies gestattet es, einen Presssitz zwischen den Gehäusebauteilen 42, 44 vorzusehen, welcher mit geringem Kraftaufwand hergestellt werden kann, oder auf den Presssitz ganz zu verzichten und eine rein axiale Abdichtung zwischen den Gehäusebauteilen 42, 44 herzustellen. Hierdurch wird der Aufwand in der maschinellen Fertigung reduziert.

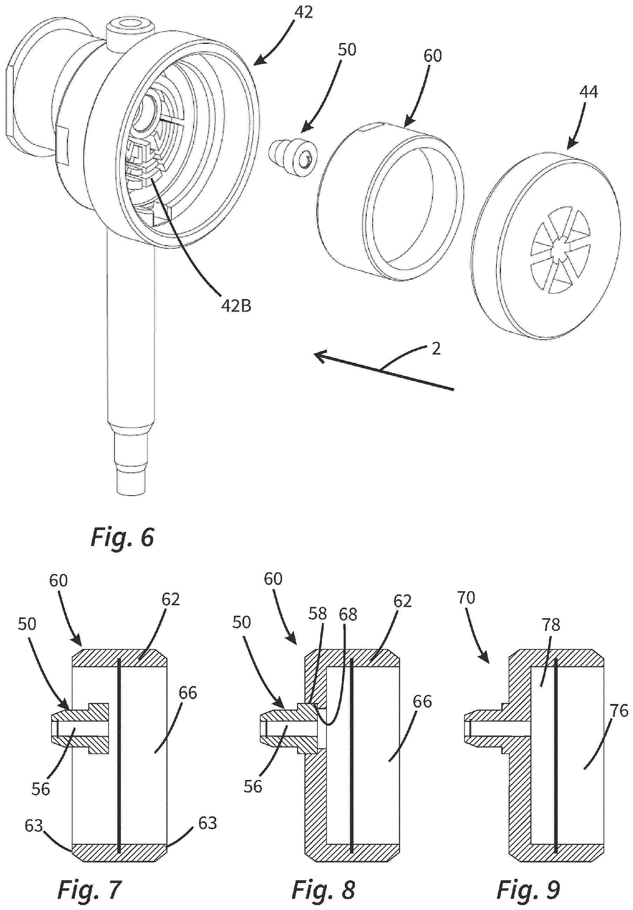

Die Fig. 6 zeigt nochmals in einer Explosionsdarstellung die inneren Bestandteile des Austragkopfes, also das erste Gehäusebauteil 42, welches gleichzeitig das Hauptbauteil des Austragkopfes 40 darstellt, sowie die Düseneinheit 50, die Filtereinheit 60 und das zweite Gehäusebauteil 44, die nacheinander in gleicher Montagerichtung 2 in das Gehäusebauteil 42 eingeschoben werden. Zusätzlich ist in der Fig. 5 auch zu erkennen, dass im Gehäusebauteil 42 eine Stützstruktur 42b vorgesehen ist, die eine zu weite Auslenkung der Filtermembran 64 und somit eine Beschädigung derselben durch zu weite Auslenkung verhindert.

Die Fig. 8 und 9 zeigen im Kontrast zur Darstellung der Fig. 7 alternative Gestaltungen der Düseneinheit 50 und der Filtereinheit 60. Bei der Darstellung der Fig. 8 ist die Bauform der Düseneinheit 50 nahezu identisch mit dem vorangegangenen Ausführungsbeispiel. Allerdings ist hier die Filtereinheit 60 und insbesondere deren Hülsenbauteil 62 anders geartet, so dass die Filtereinheit 60 und die Düseneinheit 50 mittels Steckabschnitten 58, 68 zu einer vormontierten Filter- und Düseneinheit zusammensteckt werden können. Dies kann logistisch in der Fertigung ein Vorteil sein, da der Hersteller der Düseneinheiten 50 und Filtereinheiten 60 diese bereits zusammenfügen kann, so dass der Hersteller des Austragkopfes 40 bzw. des Flüssigkeitsspenders 10 nur noch die entstandene vormontierte Baugruppe zu montieren hat. Bei der Ausgestaltung der Fig. 9 ist eine gemeinsame Filter- und Düseneinheit 70 vorgesehen, die ein gemeinsames einstückiges Hülsenbauteil 72 aufweist. Dies wird grundsätzlich als nachteilig angesehen, da die Flexibilität bei der Auswahl der passenden Filtermembran in Abhängigkeit des Anwendungszwecks entfällt. Dennoch wird auch hier eine sehr einfach zu montierende Vormontageeinheit geschaffen. Wichtig ist hierbei, dass auch hier die wirksame Filterfläche der Filtermembran frei durchströmbar bleibt. Daher ist zwischen der Filtermembran und der Düsenplatte ein Abschnitt vorgesehen, innerhalb dessen der das Hülsenbauteil durchdringende Austragkanal noch einen großen Durchmesser aufweist, bevor er an einer Sprungstelle des Gehäuses auf einen Durchmesser verjüngt wird, der in etwa dem der Düsenöffnungsfläche der Düsenplatte entspricht.

## Patentansprüche

1. Sprühanordnung, insbesondere zum Austrag von pharmazeutischen oder kosmetischen Flüssigkeiten, mit den folgenden Merkmalen:
a. die Sprühanordnung umfasst einen Zuführkanal (42A) zur Zuführung von Flüssigkeit zum Zwecke des Versprühens, und
b. die Sprühanordnung umfasst eine Düsenplatte (54), durch die hindurch Flüssigkeit vom Zuführkanal (42A) in eine umgebende Atmosphäre ausgetragen werden kann, und
c. die Düsenplatte (54) weist eine Vielzahl von Düsenöffnungen (54B) auf, deren Durchmesser maximal 15 µm beträgt und die gemeinsam eine Düsenöffnungsfläche (54A) der Düsenplatte (54) definieren,
**gekennzeichnet durch** die folgenden Merkmale:
d. die Sprühanordnung umfasst in einem Strömungspfad (4) zwischen dem Zuführkanal (42A) und der Düsenplatte (54) eine Filtermembran (64), deren Trenngrenze kleiner als der Durchmesser der Düsenöffnungen (54b) ist und deren im Strömungspfad (4) gelegene wirksame Filterfläche (64a) mindestens Faktor 20 größer ist als die Düsenöffnungsfläche (54a).

2. Sprühanordnung nach Anspruch 1 mit mindestens einem derfolgenden Merkmale:
a. die Düsenplatte (54) besteht aus einem Siliziumwerkstoff, und/oder
b. die Düsenplatte (54) weist insbesondere für pharmazeutische Anwendungen mindestens 10 Düsenöffnungen (54B) auf, vorzugsweise mindestens 20 Düsenöffnungen (54B), und/oder
c. die Düsenplatte (54) weist insbesondere für kosmetische Anwendungen mindestens 50 Düsenöffnungen (54B) auf, vorzugsweise mindestens 200 Düsenöffnungen (54B).

3. Sprühanordnung nach Anspruch 1 oder 2 mit mindestens einem der folgenden Merkmale:
a. Die Filtermembran (64) weist eine Trenngrenze von maximal 10 µm auf, vorzugsweise von maximal 5 µm, weiterhin vorzugsweise von maximal 2 µm auf, insbesondere vorzugsweise von maximal 1 µm oder von maximal 0,2 µm, und/oder
b. die wirksame Filterfläche (64A) ist mindestens Faktor 50 größer als die Düsenöffnungsfläche (54A), vorzugsweise mindestens Faktor 100 größer, insbesondere vorzugweise mindestens Faktor 200 größer und/oder
c. die Düsenplatte (54) und die Filtermembran (64) sind parallel zueinander angeordnet, und/oder
d. die Dicke der Filtermembran (64) beträgt maximal 10% der minimalen Erstreckung der wirksamen Filterfläche (64A) und/oder
e. die Filtermembran (64) ist aus einem der folgenden Werkstoffe gefertigt: Polysulfon, Polyethersulfon, Cellulose, Celluloseester, Silikone, Polyamid, Polyamidimid, Polyamid Harnstoff, Polycarbonat, Keramik, Edelstahl, Silber, Silizium, Zeolithe, Polyacrylnitril, Polyethylen, Polypropylen, Polytetrafluorethylen, Polyvinylidenfluorid, Polyvinylchlorid, Polypiperazinamid.

4. Austragkopf (40) für einen Flüssigkeitsspender (10) zum Austrag von pharmazeutischen oder kosmetischen Flüssigkeiten mit den folgenden Merkmalen:
a. der Austragkopf (40) umfasst eine Sprühanordnung nach einem der vorstehenden Ansprüche.

5. Austragkopf (40) nach Anspruch 4 mit dem folgenden Merkmal:
a. die Düsenplatte (54) und die Filtermembran (64) sind in einer gemeinsamen Filter- und Düseneinheit (70) zusammengefasst, die ein Hülsenbauteil (72) umfasst, welches von einem Austragkanal (76) durchdrungen ist, innerhalb dessen die Düsenplatte (54) und die Filtermembran (64) angeordnet sind.

6. Austragkopf (40) nach Anspruch 4 mit den folgenden Merkmalen:
a. die Düsenplatte (54) ist Teil einer Düseneinheit (50), die ein Hülsenbauteil (52) umfasst, welches von einem Austragkanal (56) durchdrungen ist, innerhalb dessen die Düsenplatte (54) angeordnet ist, und
b. die Filtermembran (64) ist Teil einer von der Düseneinheit (50) separaten Filtereinheit (60), die ein Hülsenbauteil (62) umfasst, welches von einem Austragkanal (66) durchdrungen ist, innerhalb dessen die Filtermembran (64) angeordnet ist.

7. Austragkopf (40) nach Anspruch 6 mit dem folgenden Merkmal:
a. die Düseneinheit (50) und die Filtereinheit (60) verfügen über zusammenwirkende Kopplungsabschnitte (58, 68), so dass sie zu einer gemeinsam handhabbaren Düsen- und Filtereinheit zusammengefügt werden können.

8. Austragkopf (40) nach Anspruch 6 oder 7 mit den folgenden Merkmalen:
a. der Austragkopf (40) verfügt über ein Gehäusebauteil (42) mit Aufnahmebereichen (42C, 42D) für die Düseneinheit (50) und die Filtereinheit (60), und
b. die Aufnahmebereiche (42C, 42D) sind zum Einsetzen der Düseneinheit (50) und der Filtereinheit (60) in übereinstimmender Montagerichtung (2) ausgebildet.

9. Austragkopf (40) nach einem der Ansprüche 6 bis 8 mit dem folgenden Merkmal:
a. die Hülsenbauteile (52, 62) der Filtereinheit (60) und der Düseneinheit (50) sind derart aufeinander abgestimmt, dass eines der Hülsenbauteile (52, 62) zumindest teilweise innerhalb des anderen Hülsenbauteils (62, 52) angeordnet werden kann.

10. Austragkopf (40) nach einem der Ansprüche 4 bis 9 mit dem folgenden Merkmal:
a. der Austragkopf (40) verfügt über eine Stützstruktur (42B) stromabwärts der Filtermembran (64), durch die die Auslenkung der Filtermembran (64) limitiert ist.

11. Austragkopf (40) nach einem der Ansprüche 4 bis 10 mit mindestens einem der folgenden Merkmale:
a. das Hülsenbauteil (52) der Düseneinheit (50) und/oder das Hülsenbauteil (62) der Filtereinheit (60) oder das Hülsenbauteil (72) der gemeinsamen Filter- und Düseneinheit (70) ist als Kunststoffteil ausgebildet und/oder
b. die Filtermembran (64) ist vom Hülsenbauteil (62, 72) der Filtereinheit (60) oder der gemeinsamen Filter- und Düseneinheit (70) umspritzt, und/oder
c. die Düseneinheit (50) und die Filtereinheit (60) sind in ein erstes Gehäusebauteil (42) eingesetzt, welches mit einem zweiten Gehäusebauteil (44) in mit Montagerichtung (2) der Düseneinheit (50) und der Filtereinheit (60) übereinstimmender Montagerichtung (2) verschlossen ist, und/oder
d. der Austragkopf (40) verfügt über ein Außengehäuse (46), in welches das erste und das zweite Gehäusebauteil (42, 44) gemeinsam eingeschoben werde können und welches dadurch die beiden Gehäuseteile (42, 44) zusammendrückt oder einer Trennung der beiden Gehäuseteil (42,44) entgegenwirkt, und/oder
e. die Filtereinheit (60) verfügt über einen Schutzrand (63), der derart an die Geometrie der Filtereinheit (60) angepasst ist, dass es bei zwei baugleichen Filtereinheiten (60) nicht möglich ist, eine Oberfläche der Filtermembran (64) der einen Filtereinheit (60) mit der anderen Filtereinheit (60) zu berühren.

12. Flüssigkeitsspender (10) zum Austrag von pharmazeutischen oder kosmetischen Flüssigkeiten mit den folgenden Merkmalen:
a. der Flüssigkeitsspender (10) umfasst einen Flüssigkeitsspeicher (20) zur Lagerung von Flüssigkeit vor dem Versprühen, und
b. der Flüssigkeitsspender (10) umfasst einen Austragkopf (40) nach einem der Ansprüche 4 bis 11, wobei der Zuführkanal (42A) über eine Fördereinrichtung (30) in Art einer Pumpe oder eines Auslassventils mit dem Flüssigkeitsspeicher verbunden ist.

13. Flüssigkeitsspender (10) nach Anspruch 12 mit mindestens einem der folgenden Merkmale:
a. der Flüssigkeitsspeicher (20) ist zur Aufnahme von maximal 500 ml ausgelegt, vorzugsweise von maximal 250 ml und/oder
b. der Flüssigkeitsspeicher (20) ist befüllt mit:
- einer salzhaltigen wässrigen Lösung oder
- einer wässrigen Lösung in Form einer Ringerlösung oder einer gepufferten Lösung oder
- einer wässrigen Lösung mit mindestens einem der Zusätze Kohlenhydrate, ätherische
- Öle, Menthol und Pflanzenextrakte oder
- einer wässrigen Lösung, enthaltend Vitamine, Spurenelemente, Mangan oder Zink, oder
- einer wässrigen Lösung mit mindestens einem der Zusätze aus der Gruppe umfassend Zimtöl, Teebaumöl, Salbeiöl, Thymianöl, Zitronenmelissenöl.

14. Flüssigkeitsspender (10) nach einem der Ansprüche 12 oder 13 mit dem folgenden Merkmal:
a. der Flüssigkeitsspender (10) ist als Inhalator ausgebildet und verfügt über ein Atemstück (12), welches als Mundstück oder als Atemmaske ausgestaltet ist, und/oder
b. das Atemstück (12) weist einen Kanalabschnitt auf, der in einer von einer Betätigungsrichtung des Austragskopfs abweichenden Richtung ausgerichtet ist und vorzugsweise mit dieser einen Winkel zwischen 45° und 135° einschließt, insbesondere vorzugsweise einen Winkel zwischen 70° und 110°, und/oder
c. der Flüssigkeitsspeicher (20) ist als Druckspeicher ausgebildet.

## Claims

1. Spray assembly, in particular for discharging pharmaceutical or cosmetic liquids, with the following features:
a. the spray assembly comprises a feed channel (42A) for feeding liquid for the purpose of spraying, and
b. the spray assembly comprises a nozzle plate (54), through which liquid can be discharged from the feed channel (42A) into the surrounding atmosphere, and
c. the nozzle plate (54) has a multiplicity of nozzle openings (54B), the diameter of which is a maximum of 15 µm and which together define a nozzle opening area (54A) of the nozzle plate (54),
**characterized by** the following features:
d. the spray assembly comprises in a flow path (4) between the feed channel (42A) and the nozzle plate (54) a filter membrane (64), the separation limit of which is less than the diameter of the nozzle openings (54B) and the effective filter area (64a) of which in the flow path (4) is greater by a factor of at least 20 than the nozzle opening area (54a).

2. Spray assembly according to Claim 1 with at least one of the following features:
a. the nozzle plate (54) consists of a silicon material, and/or
b. in particular for pharmaceutical applications, the nozzle plate (54) has at least 10 nozzle openings (54B), preferably at least 20 nozzle openings (54B), and/or
c. in particular for cosmetic applications, the nozzle plate (54) has at least 50 nozzle openings (54B), preferably at least 200 nozzle openings (54B) .

3. Spray assembly according to Claim 1 or 2 with at least one of the following features:
a. the filter membrane (64) has a separation limit of a maximum of 10 µm, preferably a maximum of 5 µm, more preferably a maximum of 2 µm, in particular preferably a maximum of 1 µm or a maximum of 0.2 µm, and/or
b. the effective filter area (64A) is greater by a factor of at least 50 than the nozzle opening area (54A), preferably greater by a factor of at least 100, in particular preferably greater by a factor of at least 200, and/or
c. the nozzle plate (54) and the filter membrane (64) are arranged parallel to one another, and/or
d. the thickness of the filter membrane (64) is a maximum of 10% of the minimum extent of the effective filter area (64A), and/or
e. the filter membrane (64) is produced from one of the following materials: polysulfone, polyethersulfone, cellulose, cellulose ester, silicones, polyamide, polyamide imide, polyamide urea, polycarbonate, ceramic, stainless steel, silver, silicon, zeolites, polyacrylonitrile, polyethylene, polypropylene, polytetrafluoroethylene, polyvinylidene fluoride, polyvinylchloride, poly piperazine amide.

4. Discharge head (40) for a liquid dispenser (10) for discharging pharmaceutical or cosmetic liquids with the following features:
a. the discharge head (40) comprises a spray assembly according to one of the preceding claims.

5. Discharge head (40) according to Claim 4 with the following feature:
a. the nozzle plate (54) and the filter membrane (64) are combined in a common filter and nozzle unit (70), which comprises a sleeve component (72), which is penetrated by a discharge channel (76), within which the nozzle plate (54) and the filter membrane (64) are arranged.

6. Discharge head (40) according to Claim 4 with the following features:
a. the nozzle plate (54) is part of a nozzle unit (50), which comprises a sleeve component (52), which is penetrated by a discharge channel (56), within which the nozzle plate (54) is arranged, and
b. the filter membrane (64) is part of a filter unit (60) that is separate from the nozzle unit (50) and comprises a sleeve component (62), which is penetrated by a discharge channel (66), within which the filter membrane (64) is arranged.

7. Discharge head (40) according to Claim 6 with the following feature:
a. the nozzle unit (50) and the filter unit (60) have interacting coupling portions (58, 68), so that they can be joined together to form a nozzle and filter unit that can be handled together.

8. Discharge head (40) according to Claim 6 or 7 with the following features:
a. the discharge head (40) has a housing component (42) with receiving regions (42C, 42D) for the nozzle unit (50) and the filter unit (60), and
b. the receiving regions (42C, 42D) are formed for the insertion of the nozzle unit (50) and the filter unit (60) in a coinciding mounting direction (2).

9. Discharge head (40) according to one of Claims 6 to 8 with the following feature:
a. the sleeve components (52, 62) of the filter unit (60) and of the nozzle unit (50) are made to match one another in such a way that one of the sleeve components (52, 62) can be arranged at least partially within the other sleeve component (62, 52) .

10. Discharge head (40) according to one of Claims 4 to 9 with the following feature:
a. the discharge head (40) has downstream of the filter membrane (64) a supporting structure (42B), by which the deflection of the filter membrane (64) is limited.

11. Discharge head (40) according to one of Claims 4 to 10 with at least one of the following features:
a. the sleeve component (52) of the nozzle unit (50) and/or the sleeve component (62) of the filter unit (60) or the sleeve component (72) of the common filter and nozzle unit (70) is formed as a part made of plastics and/or
b. the filter membrane (64) is encapsulated by the sleeve component (62, 72) of the filter unit (60) or of the common filter and nozzle unit (70), and/or
c. the nozzle unit (50) and the filter unit (60) are inserted in a first housing component (42), which is closed by a second housing component (44) in a mounting direction (2) coinciding with the mounting direction (2) of the nozzle unit (50) and of the filter unit (60), and/or
d. the discharge head (40) has an outer housing (46), into which the first and second housing components (42, 44) can be jointly pushed and which as a result presses together the two housing components (42, 44) or counteracts a separation of the two housing components (42, 44), and/or
e. the filter unit (60) has a guard rim (63), which is adapted to the geometry of the filter unit (60) in such a way that, when there are two filter units (60) of the same type, it is not possible for one surface of the filter membrane (64) of the one filter unit (60) to come into contact with the other filter unit (60).

12. Liquid dispenser (10) for discharging pharmaceutical or cosmetic liquids with the following features:
a. the liquid dispenser (10) comprises a liquid reservoir (20) for storing liquid before spraying, and
b. the liquid dispenser (10) comprises a discharge head (40) according to one of Claims 4 to 11, the feed channel (42A) being connected to the liquid reservoir by way of a delivery device (30) in the manner of a pump or an outlet valve.

13. Liquid dispenser (10) according to Claim 12 with at least one of the following features:
a. the liquid reservoir (20) is designed for receiving a maximum of 500 ml, preferably a maximum of 250 ml and/or
b. the liquid reservoir (20) is filled with:
- a saline aqueous solution or
- an aqueous solution in the form of a Ringer's solution or a buffered solution or
- an aqueous solution with at least one of the additives carbohydrates, essential oils, menthol and plant extracts or
- an aqueous solution containing vitamins, trace elements, manganese or zinc, or
- an aqueous solution with at least one of the additives from the group comprising cinnamon oil, tea tree oil, sage oil, thyme oil and lemon balm oil.

14. Liquid dispenser (10) according to either of Claims 12 and 13 with the following feature:
a. the liquid dispenser (10) is formed as an inhaler and has a respiration piece (12), which is configured as a mouthpiece or as a breathing mask, and/or
b. the respiration piece (12) has a channel portion which is oriented in a direction deviating from an actuation direction of the discharge head and which preferably encloses with the latter an angle of between 45° and 135°, in particular preferably an angle of between 70° and 110°, and/or
c. the liquid reservoir (20) is formed as a pressure reservoir.

## Revendications

1. Système de pulvérisation, en particulier pour distribuer des liquides pharmaceutiques ou cosmétiques, comprenant les caractéristiques suivantes :
a. le système de pulvérisation comprend un canal d'alimentation (42A) pour alimenter du liquide à pulvériser, et
b. le système de pulvérisation comprend une plaque à buses (54) à travers laquelle du liquide peut être distribué depuis le canal d'alimentation (42A) dans une atmosphère environnante, et
c. la plaque à buses (54) présente une pluralité d'ouvertures de buses (54B) dont le diamètre vaut au maximum 15 µm et qui définissent en commun une surface d'ouverture de buse (54A) de la plaque à buses (54),
**caractérisé par** les caractéristiques suivantes :
d. le système de pulvérisation comprend une membrane de filtre (64) dans un chemin d'écoulement (4) entre le canal d'alimentation (42A) et la plaque à buses (54), dont la limite de séparation est inférieure au diamètre des ouvertures de buses (54b) et dont la surface de filtre active (64a) située dans le chemin d'écoulement (4) est plus grande d'au moins un facteur 20 que la surface d'ouverture de buse (54a).

2. Système de pulvérisation selon la revendication 1, comprenant au moins l'une des caractéristiques suivantes :
a. la plaque à buses (54) se compose d'un matériau à base de silicium et/ou
b. la plaque à buses (54) présente, notamment pour des applications pharmaceutiques, au moins 10 ouvertures de buses (54B), de préférence au moins 20 ouvertures de buses (54B), et/ou
c. la plaque à buses (54) présente, notamment pour des applications cosmétiques, au moins 50 ouvertures de buses (54B), de préférence au moins 200 ouvertures de buses (54B).

3. Système de pulvérisation selon la revendication 1 ou 2, comprenant au moins l'une des caractéristiques suivantes :
a. la membrane de filtre (64) présente une limite de séparation de 10 *µm* au maximum, de préférence de 5 *µm* au maximum, de préférence en outre de 2 *µm* au maximum, en particulier de préférence de 1 *µm* au maximum ou de 0,2 *µm* au maximum, et/ou
b. la surface de filtre active (64A) est plus grande que la surface d'ouverture de buses (54A) d'au moins un facteur 50, de préférence d'au moins un facteur 100, particulièrement préférablement d'au moins un facteur 200 et/ou
c. la plaque à buses (54) et la membrane de filtre (64) sont disposées parallèlement l'une à l'autre, et/ou
d. l'épaisseur de la membrane de filtre (64) vaut au maximum 10 % de l'étendue minimale de la surface de filtre active (64A) et/ou
e. la membrane de filtre (64) est fabriquée à partir d'un des matériaux suivants : polysulfone, polyéthersulfone, cellulose, ester de cellulose, silicone, polyamide, polyamide imide, polyamide-urée, polycarbonate, céramique, acier inoxydable, argent, silicium, zéolithes, polyacrylonitrile, polyéthylène, polypropylène, polytétrafluoroéthylène, fluorure de polyvinylidène, chlorure de polyvinyle, polypipérazinamide.

4. Tête de distribution (40) pour un distributeur de liquide (10) pour distribuer des liquides pharmaceutiques ou cosmétiques, comprenant les caractéristiques suivantes :
a. la tête de distribution (40) comprend un système de pulvérisation selon l'une quelconque des revendications précédentes.

5. Tête de distribution (40) selon la revendication 4, comprenant la caractéristique suivante :
a. la plaque à buses (54) et la membrane de filtre (64) sont réunies en une unité de filtre et de buse commune (70), qui comprend un composant de douille (72) qui est traversé par un canal de distribution (76) à l'intérieur duquel sont disposées la plaque à buses (54) et la membrane de filtre (64).

6. Tête de distribution (40) selon la revendication 4, comprenant les caractéristiques suivantes :
a. la plaque à buses (54) fait partie d'une unité de buse (50) qui comprend un composant de douille (52) qui est traversé par un canal de distribution (56) à l'intérieur duquel est disposée la plaque à buses (54), et
b. la membrane de filtre (64) fait partie d'une unité de filtre (60) séparée de l'unité de buse (50), qui comprend un composant de douille (62) qui est traversé par un canal de distribution (66) à l'intérieur duquel est disposée la membrane de filtre (64).

7. Tête de distribution (40) selon la revendication 6, comprenant la caractéristique suivante :
a. l'unité de buse (50) et l'unité de filtre (60) disposent de portions d'accouplement coopérantes (58, 68) de telle sorte qu'elles puissent être assemblées pour former une unité de buse et de filtre pouvant être manipulée en commun.

8. Tête de distribution (40) selon la revendication 6 ou 7, comprenant les caractéristiques suivantes :
a. la tête de distribution (40) dispose d'un composant de boîtier (42) avec des régions de réception (42C, 42D) pour l'unité de buse (50) et l'unité de filtre (60), et
b. les régions de réception (42C, 42D) sont réalisées pour l'insertion de l'unité de buse (50) et de l'unité de filtre (60) dans une direction de montage (2) coïncidente.

9. Tête de distribution (40) selon l'une quelconque des revendications 6 à 8, comprenant la caractéristique suivante :
a. les composants de douille (52, 62) de l'unité de filtre (60) et de l'unité de buse (50) sont ajustés l'un à l'autre de telle sorte que l'un des composants de douille (52, 62) puisse être disposé au moins en partie à l'intérieur de l'autre composant de douille (62, 52).

10. Tête de distribution (40) selon l'une quelconque des revendications 4 à 9, comprenant la caractéristique suivante :
a. la tête de distribution (40) dispose d'une structure de support (42B) en aval de la membrane de filtre (64), par laquelle la déviation de la membrane de filtre (64) est limitée.

11. Tête de distribution (40) selon l'une quelconque des revendications 4 à 10, comprenant au moins l'une des caractéristiques suivantes :
a. le composant de douille (52) de l'unité de buse (50) et/ou le composant de douille (62) de l'unité de filtre (60) ou le composant de douille (72) de l'unité de filtre et de buse commune (70) est réalisé sous forme de pièce en plastique et/ou
b. la membrane de filtre (64) est surmoulée par le composant de douille (62, 72) de l'unité de filtre (60) ou de l'unité de filtre et de buse commune (70), et/ou
c. l'unité de buse (50) et l'unité de filtre (60) sont insérées dans un premier composant de boîtier (42) qui est fermé avec un deuxième composant de boîtier (44) dans une direction de montage (2) coïncidant avec la direction de montage (2) de l'unité de buse (50) et de l'unité de filtre (60), et/ou
d. la tête de distribution (40) dispose d'un boîtier extérieur (46) dans lequel le premier et le deuxième composant de boîtier (42, 44) peuvent être enfoncés en commun et qui comprime de ce fait les deux composants de boîtier (42, 44) ou s'oppose à une séparation des deux composants de boîtier (42, 44), et/ou
e. l'unité de filtre (60) dispose d'un bord de protection (63) qui est adapté à la géométrie de l'unité de filtre (60) de telle sorte que lorsque deux unités de filtre sont de construction identique (60), il ne soit pas possible qu'une surface de la membrane de filtre (64) d'une unité de filtre (60) vienne en contact avec l'autre unité de filtre (60).

12. Distributeur de liquide (10) pour distribuer des liquides pharmaceutiques ou cosmétiques, comprenant les caractéristiques suivantes :
a. le distributeur de liquide (10) comprend un réservoir de liquide (20) pour stocker du liquide avant sa pulvérisation, et
b. le distributeur de liquide (10) comprend une tête de distribution (40) selon l'une quelconque des revendications 4 à 11, le canal d'alimentation (42A) étant connecté au distributeur de liquide par le biais d'un dispositif de refoulement (30) de type pompe ou soupape de sortie.

13. Distributeur de liquide (10) selon la revendication 12, comprenant au moins l'une des caractéristiques suivantes :
a. le réservoir de liquide (20) est conçu pour recevoir au maximum 500 ml, de préférence au maximum 250 ml et/ou
b. le réservoir de liquide (20) est rempli avec :
- une solution aqueuse salée ou
- une solution aqueuse sous la forme d'une solution de Ringer ou d'une solution tamponnée ou
- une solution aqueuse avec au moins l'un des additifs hydrates de carbone, huiles essentielles, menthol et extraits végétaux ou
- une solution aqueuse contenant des vitamines, des oligo-éléments, du manganèse ou du zinc, ou
- une solution aqueuse comprenant au moins l'un des additifs appartenant au groupe comprenant l'huile de cannelle, l'huile de l'arbre à thé, l'huile de sauge, l'huile de thym, l'huile de mélisse officinale.

14. Distributeur de liquide (10) selon l'une quelconque des revendications 12 et 13, comprenant la caractéristique suivante :
a. le distributeur de liquide (10) est réalisé sous forme d'inhalateur et dispose d'une pièce respiratoire (12) qui est configurée sous forme d'embout ou de masque respiratoire, et/ou
b. la pièce respiratoire (12) présente une portion de canal qui est orientée dans une direction s'écartant de la direction d'actionnement de la tête de distribution et forme de préférence avec celle-ci un angle compris entre 45° et 130°, notamment de préférence un angle compris entre 70° et 110°, et/ou
c. le réservoir de liquide (20) est réalisé sous forme d'accumulateur de pression.
